# EUROPEAN PATENT APPLICATION

(11) **EP 4 831 634 A1**
(43) Date of publication of application: **28.10.2026**
(21) Application number: 24907458.4
(22) Date of filing: 18.12.2024
(51) Int. Cl.: G01N 33/543, C07K 16/00, C07K 16/46, C07K 17/08, G01N 33/531

(54) **METHOD FOR MEASURING TARGET ANTIGEN, AND ANTIBODY, INSOLUBLE PARTICLES AND TARGET ANTIGEN MEASUREMENT KIT USED IN SAID METHOD**

(30) Priority: 18.12.2023 JP 2023212927
(71) Applicant: Denka Company Limited, Tokyo 103-8338 (JP)
(72) Inventor: YOSHIZAKI, Kyohei, Tokyo 103-8338 (JP); IKENAGA, Yasuyuki, Tokyo 103-8338 (JP); HONDA, Takumi, Tokyo 103-8338 (JP)
(74) Representative: Hoffmann Eitle
(86) International application number: PCT/JP2024/044806
(87) International publication number: WO 2025/135074

(57) **Abstract**

An IgG antibody, other than human IgG, having a Fab region and an Fc region connected to the Fab region, wherein four or more N-glycans are bound to the Fc region.

## Description

### TECHNICAL FIELD

The present invention relates to a target antigen measurement method, and to an antibody, insoluble particles, and a target antigen measurement kit used in said method.

### BACKGROUND

As target antigen measurement methods making use of immune reactions, there are methods making use of particulTabate carriers such as latex (for example, Patent Document 1). Particulate carriers to which antibodies that are specific to a target antigen are bound will undergo antigen-antibody reactions if the target antigen is present. Furthermore, due to the specificity and the binding affinity thereof, the target antigen will serve as a bridge for mutual binding and agglutination. The presence or the amount of the target antigen is measured by whether or not agglutinated clumps have been created or by the degree of agglutination.

Such measurement methods making use of immune reactions sometimes have problems such as insufficient detection sensitivity. For example, Patent Document 2 discloses polysaccharide-modified complex particles in which the occurrence of non-specific adsorption is reduced so that the measurement sensitivity will not become lower.

### CITATION LIST

### PATENT LITERATURE

Patent Document 1: JP 2017-83440 A
Patent Document 2: JP 2009-162537 A

### SUMMARY OF INVENTION

### TECHNICAL PROBLEM

However, since the above-mentioned polysaccharide-modified complex particles are produced by making use of polysaccharides adsorbing to the surfaces of silica particles, the types of polysaccharide-modified complex particles were limited and the uses thereof were restricted.

In consideration of the above-mentioned circumstances, an objective of the present invention is to provide an antibody that, when carried on a particulate carrier, can suppress the non-specific agglutination of insoluble particles containing said particulate carrier, the insoluble particles containing said antibody, a target antigen measurement kit using said antibody or said insoluble particles, or a target antigen measurement method using said insoluble particles.

### SOLUTION TO PROBLEM

Upon performing diligent research, the present inventors discovered that an insoluble particle containing a particulate carrier carrying an IgG antibody, wherein the IgG antibody has a Fab region and an Fc region connected to the Fab region, and four or more glycans are bound to the Fc region of the IgG antibody can suppress non-specific agglutination, thereby completing the present invention.

The present invention provides, for example, the respective inventions indicated below.
[1] An IgG antibody, other than human IgG, having a Fab region and an Fc region connected to the Fab region, wherein four or more N-glycans are bound to the Fc region.
[2] The antibody according to [1], to which four N-glycans are bound.
[3] The antibody according to [1] or [2], wherein two N-glycans are bound to a second constant domain and two N-glycans are bound to a third constant domain.
[3a] The antibody according to [1] or [2], wherein two N-glycans among the four or more N-glycans are bound to a second constant domain, and two N-glycans different from the aforementioned two N-glycans among the four or more N-glycans are bound to a third constant domain.
[4] The antibody according to any one of [1] to [3a], wherein an amino acid sequence of the Fc region:
   (1) has 90% or higher identity with an amino acid sequence of SEQ ID NO: 2, and in the amino acid sequence,
      a 174-th residue is an asparagine residue, a 175-th residue is an amino acid residue other than proline, a 176-th residue is a serine residue or a threonine residue, a 301-st residue is an asparagine residue, a 302-nd residue is an amino acid residue other than proline, and a 303-rd residue is a serine residue or a threonine residue;
   (2) has 90% or higher identity with an amino acid sequence of SEQ ID NO: 4, and in the amino acid sequence,
      a 180-th residue is an asparagine residue, a 181-st residue is an amino acid residue other than proline, a 182-nd residue is a serine residue or a threonine residue, a 307-th residue is an asparagine residue, a 308-th residue is an amino acid residue other than proline, and a 309-th residue is a serine residue or a threonine residue;
   (3) has 90% or higher identity with an amino acid sequence of SEQ ID NO: 6, and in the amino acid sequence,
      a 185-th residue is an asparagine residue, a 186-th residue is an amino acid residue other than proline, a 187-th residue is a serine residue or a threonine residue, a 312-th residue is an asparagine residue, a 313-th residue is an amino acid residue other than proline, and a 314-th residue is a serine residue or a threonine residue;
   (4) has 90% or higher identity with an amino acid sequence of SEQ ID NO: 8, and in the amino acid sequence,
      a 179-th residue is an asparagine residue, a 180-th residue is an amino acid residue other than proline, a 181-st residue is a serine residue or a threonine residue, a 306-th residue is an asparagine residue, a 307-th residue is an amino acid residue other than proline, and a 308-th residue is a serine residue or a threonine residue; or
   (5) has 90% or higher identity with an amino acid sequence of SEQ ID NO: 16, and in the amino acid sequence,
   a 173-rd residue is an asparagine residue, a 174-th residue is an amino acid residue other than proline, a 175-th residue is a serine residue or a threonine residue, a 300-th residue is an asparagine residue, a 301-st residue is an amino acid residue other than proline, and a 302-nd residue is a serine residue or a threonine residue.
[5] An insoluble particle containing a particulate carrier and an IgG antibody to a target antigen, the IgG antibody being carried on the particulate carrier, wherein:
   the antibody has a Fab region and an Fc region connected to the Fab region; and
   four or more N-glycans are bound to the Fc region.
[6] The insoluble particle according to [5], wherein four N-glycans are bound to the antibody.
[7] The insoluble particle according to [5] or [6], wherein, in the antibody, two N-glycans are bound to a second constant domain, and two N-glycans are bound to a third constant domain.
[7a] The insoluble particle according to [5] or [6], wherein, in the antibody, two N-glycans among the four or more N-glycans are bound to a second constant domain, and two N-glycans different from the aforementioned two N-glycans among the four or more N-glycans are bound to a third constant domain.
[8] The insoluble particle according to any one of [5] to [7a], wherein the particulate carrier is a latex particle.
[9] The insoluble particle according to any one of [5] to [8], wherein an amino acid sequence of the Fc region in the antibody:
   (1) has 90% or higher identity with an amino acid sequence of SEQ ID NO: 2, and in the amino acid sequence,
      a 174-th residue is an asparagine residue, a 175-th residue is an amino acid residue other than proline, a 176-th residue is a serine residue or a threonine residue, a 301-st residue is an asparagine residue, a 302-nd residue is an amino acid residue other than proline, and a 303-rd residue is a serine residue or a threonine residue;
   (2) has 90% or higher identity with an amino acid sequence of SEQ ID NO: 4, and in the amino acid sequence,
      a 180-th residue is an asparagine residue, a 181-st residue is an amino acid residue other than proline, a 182-nd residue is a serine residue or a threonine residue, a 307-th residue is an asparagine residue, a 308-th residue is an amino acid residue other than proline, and a 309-th residue is a serine residue or a threonine residue;
   (3) has 90% or higher identity with an amino acid sequence of SEQ ID NO: 6, and in the amino acid sequence,
      a 185-th residue is an asparagine residue, a 186-th residue is an amino acid residue other than proline, a 187-th residue is a serine residue or a threonine residue, a 312-th residue is an asparagine residue, a 313-th residue is an amino acid residue other than proline, and a 314-th residue is a serine residue or a threonine residue;
   (4) has 90% or higher identity with an amino acid sequence of SEQ ID NO: 8, and in the amino acid sequence,
      a 179-th residue is an asparagine residue, a 180-th residue is an amino acid residue other than proline, a 181-st residue is a serine residue or a threonine residue, a 306-th residue is an asparagine residue, a 307-th residue is an amino acid residue other than proline, and a 308-th residue is a serine residue or a threonine residue;
   (5) has 90% or higher identity with an amino acid sequence of SEQ ID NO: 10, and in the amino acid sequence,
      a 180-th residue is an asparagine residue, a 181-st residue is an amino acid residue other than proline, a 182-nd residue is a serine residue or a threonine residue, a 307-th residue is an asparagine residue, a 308-th residue is an amino acid residue other than proline, and a 309-th residue is a serine residue or a threonine residue;
   (6) has 90% or higher identity with an amino acid sequence of SEQ ID NO: 12, and in the amino acid sequence,
      a 176-th residue is an asparagine residue, a 177-th residue is an amino acid residue other than proline, a 178-th residue is a serine residue or a threonine residue, a 303-rd residue is an asparagine residue, a 304-th residue is an amino acid residue other than proline, and a 305-th residue is a serine residue or a threonine residue;
   (7) has 90% or higher identity with an amino acid sequence of SEQ ID NO: 14, and in the amino acid sequence,
      a 177-th residue is an asparagine residue, a 178-th residue is an amino acid residue other than proline, a 179-th residue is a serine residue or a threonine residue, a 304-th residue is an asparagine residue, a 305-th residue is an amino acid residue other than proline, and a 306-th residue is a serine residue or a threonine residue; or
   (8) has 90% or higher identity with an amino acid sequence of SEQ ID NO: 16, and in the amino acid sequence,
   a 173-rd residue is an asparagine residue, a 174-th residue is an amino acid residue other than proline, a 175-th residue is a serine residue or a threonine residue, a 300-th residue is an asparagine residue, a 301-st residue is an amino acid residue other than proline, and a 302-nd residue is a serine residue or a threonine residue.
[10] A target antigen measurement kit including the insoluble particle according to any one of [5] to [9], or an antibody and a particulate carrier for preparing the insoluble particle according to any one of [5] to [9].
[11] A target antigen measurement method wherein the insoluble particle according to any one of [5] to [9] is used.
[12] A target antigen measurement method including:
   a step of bringing the insoluble particle according to any one of [5] to [9] into contact with a test sample that may contain a target antigen; and
   a step of measuring an agglutination reaction of the insoluble particle due to an antigen-antibody reaction between the target antigen and an antibody contained in the insoluble particle.

### EFFECTS OF INVENTION

The present invention can provide an antibody that, when carried on a particulate carrier, can suppress the non-specific agglutination of insoluble particles containing said particulate carrier, insoluble particles containing said antibody, a target antigen measurement kit using said antibody or said insoluble particles, or a target antigen measurement method using said insoluble particles.

### BRIEF DESCRIPTION OF DRAWINGS

FIG. 1 is a graph indicating the changes in absorbance by agglutination reactions of anti-human BNP antibody-sensitive latex in the presence of 0 pg/mL or 1000 pg/mL of human BNP.
FIG. 2 is a graph indicating the ratios of the change in absorbance in the presence of 1000 pg/mL of human BNP to the change in absorbance in the presence of 0 pg/mL of human BNP in FIG. 1.
FIG. 3 is a graph indicating the changes in absorbance due to non-specific agglutination reactions of anti-human BNP antibody-sensitive latex in plasma specimens not containing human BNP.
FIG. 4 is a graph indicating the results of antigen-binding activity evaluations of anti-human BNP antibodies.

### DESCRIPTION OF EMBODIMENTS

Hereinafter, the specifics of the present invention will be explained in detail.

An IgG antibody according to one embodiment has four or more N-glycans bound to the Fc region. The above-mentioned IgG antibody is not particularly restricted as long as it can specifically bind to a target antigen to be described below.

An IgG antibody according to one embodiment can suppress non-specific agglutination of insoluble particles containing particulate carriers on which the IgG antibody is carried, without compromising the antigen-binding activity of the IgG antibody.

An IgG antibody according to one embodiment may be, for example, a mouse IgG, a rabbit IgG, etc. (with the proviso that human IgG is excluded). The above-mentioned IgG antibody includes genetically modified antibodies such as an IgG antibody in which a glycan-binding motif Asn-Xaa-Ser/Thr (where Xaa is an amino acid other than proline; also referred to hereinafter as a "glycan-binding motif") has been introduced to the Fc region in the IgG antibody. Amino acids other than proline refer to amino acids selected from the group consisting of alanine, cysteine, aspartic acid, glutamic acid, phenylalanine, glycine, histidine, isoleucine, lysine, leucine, methionine, asparagine, glutamine, arginine, serine, threonine, valine, tryptophan, and tyrosine. An IgG antibody according to one embodiment is preferably one of a mouse IgG1, mouse IgG2a, mouse IgG2b, mouse IgG3, and rabbit IgG in which a glycan-binding motif has been introduced to the Fc region.

The IgG antibody has a Fab region and an Fc region connected to said Fab region. The Fab region and the Fc region in the IgG antibody may be connected by a hinge region. Additionally, the IgG antibody is constituted by two heavy chains (H chains) and two light chains (L chains). The heavy chains each have, in order from an N-terminus, a variable region (VH), a first constant domain (CH1), a second constant domain (CH2), and a third constant domain (CH3). The light chains each have, in order from an N-terminus, a variable region (VL) and a constant region (CL). The portion constituted by VH, CH1, VL, and CL is a Fab region. The portion constituted by CH2 and CH3 is an Fc region.

In an IgG antibody according to one embodiment, it is preferable for two N-glycans among four or more N-glycans bound to the Fc region to be bound to the second constant domain, and for two N-glycans different from the above-mentioned two N-glycans among the four or more N-glycans to be bound to the third constant domain.

In an IgG antibody according to one embodiment, it is preferable for four N-glycans to be bound to the Fc region, and more preferable for two N-glycans to be bound to the second constant domain and two N-glycans to be bound to the third constant domain.

In an IgG antibody according to one embodiment, it is preferable for an even number of N-glycans to be bound to the Fc region. In this case, the even number of N-glycans should preferably be respectively bound to amino acid residues that respectively correspond to each other in the Fc regions of the two heavy chains.

In an IgG antibody according to one embodiment, it is preferable for an N-glycan to be bound to an asparagine residue in a glycan-binding motif, and more preferable for all of the N-glycans to be bound to asparagine residues in glycan-binding motifs.

In an IgG antibody according to one embodiment, the Fc region in the IgG antibody has 90% or higher identity with the amino acid sequence of SEQ ID NO: 2, 4, 6, 8, or 16. Said amino acid sequences are preferably such that: in SEQ ID NO: 2, a 174-th residue is an asparagine residue, a 175-th residue is an amino acid residue other than proline, a 176-th residue is a serine residue or a threonine residue, a 301-st residue is an asparagine residue, a 302-nd residue is an amino acid residue other than proline, and a 303-rd residue is a serine residue or a threonine residue; in SEQ ID NO: 4, a 180-th residue is an asparagine residue, a 181-st residue is an amino acid residue other than proline, a 182-nd residue is a serine residue or a threonine residue, a 307-th residue is an asparagine residue, a 308-th residue is an amino acid residue other than proline, and a 309-th residue is a serine residue or a threonine residue; in SEQ ID NO: 6, a 185-th residue is an asparagine residue, a 186-th residue is an amino acid residue other than proline, a 187-th residue is a serine residue or a threonine residue, a 312-th residue is an asparagine residue, a 313-th residue is an amino acid residue other than proline, and a 314-th residue is a serine residue or a threonine residue; in SEQ ID NO: 8, a 179-th residue is an asparagine residue, a 180-th residue is an amino acid residue other than proline, a 181-st residue is a serine residue or a threonine residue, a 306-th residue is an asparagine residue, a 307-th residue is an amino acid residue other than proline, and a 308-th residue is a serine residue or a threonine residue; or in SEQ ID NO: 16, a 173-rd residue is an asparagine residue, a 174-th residue is an amino acid residue other than proline, a 175-th residue is a serine residue or a threonine residue, a 300-th residue is an asparagine residue, a 301-st residue is an amino acid residue other than proline, and a 302-nd residue is a serine residue or a threonine residue. In the preferable embodiments mentioned above, it is more preferable for glycan-binding motifs to be absent aside from the 174-th to 176-th residues and the 301-st to 303-rd residues in SEQ ID NO: 2, aside from the 180-th to 182-nd residues and the 307-th to 309-th residues in SEQ ID NO: 4, aside from the 185-th to 187-th residues and the 312-th to 314-th residues in SEQ ID NO: 6, aside from the 179-th to 181-st residues and the 306-th to 308-th residues in SEQ ID NO: 8, or aside from the 173-rd to 175-th residues and the 300-th to 302-nd residues in SEQ ID NO: 16.

In this case, SEQ ID NO: 2 is the amino acid sequence (SEQ ID NO: 1) of the Fc region in mouse IgG1 in which the threonine residue at the 301-st (i.e., the 424-th by Eu numbering) position has been substituted by an asparagine residue. In SEQ ID NOs: 1 and 2, the 174-th to 176-th residues are located in the second constant domain, and the 301-st to 303-rd residues are located in the third constant domain. SEQ ID NO: 1 has a glycan-binding motif at the 174-th to 176-th residues, and SEQ ID NO: 2 has glycan-binding motifs at the 174-th to 176-th residues and at the 301-st to 303-rd residues.

SEQ ID NO: 4 is the amino acid sequence (SEQ ID NO: 3) of the Fc region in mouse IgG2a in which the serine residue at the 307-th position has been substituted by an asparagine residue. In SEQ ID NOs: 3 and 4, the 180-th to 182-nd residues are located in the second constant domain, and the 307-th to 309-th residues are located in the third constant domain. SEQ ID NO: 3 has a glycan-binding motif at the 180-th to 182-nd residues, and SEQ ID NO: 4 has glycan-binding motifs at the 180-th to 182-nd residues and at the 307-th to 309-th residues.

SEQ ID NO: 6 is the amino acid sequence (SEQ ID NO: 5) of the Fc region in mouse IgG2b in which the serine residue at the 312-th position has been substituted by an asparagine residue, and the asparagine residue at the 314-th position has been substituted by a serine residue. In SEQ ID NOs: 5 and 6, the 185-th to 187-th residues are located in the second constant domain, and the 312-th to 314-th residues are located in the third constant domain. SEQ ID NO: 5 has a glycan-binding motif at the 185-th to 187-th residues, and SEQ ID NO: 6 has glycan-binding motifs at the 185-th to 187-th residues and at the 312-th to 314-th residues.

SEQ ID NO: 8 is the amino acid sequence (SEQ ID NO: 7) of the Fc region in mouse IgG3 in which the threonine residue at the 306-th position has been substituted by an asparagine residue. In SEQ ID NOs: 7 and 8, the 179-th to 181-st residues are located in the second constant domain, and the 306-th to 308-th residues are located in the third constant domain. SEQ ID NO: 7 has a glycan-binding motif at the 179-th to 181-st residues, and SEQ ID NO: 8 has glycan-binding motifs at the 179-th to 181-st residues and at the 306-th to 308-th residues.

SEQ ID NO: 16 is the amino acid sequence (SEQ ID NO: 15) of the Fc region in rabbit IgG in which the threonine residue at the 300-th position has been substituted by an asparagine residue. In SEQ ID NOs: 15 and 16, the 173-rd to 175-th residues are located in the second constant domain, and the 300-th to 302-nd residues are located in the third constant domain. SEQ ID NO: 15 has a glycan-binding motif at the 173-rd to 175-th residues, and SEQ ID NO: 16 has glycan-binding motifs at the 173-rd to 175-th residues and at the 300-th to 302-nd residues.

The N-glycans are not particularly restricted and may, for example, be high-mannose, hybrid, or complex N-glycans. The four or more N-glycans bound to the Fc region in an IgG antibody according to one embodiment may be one type of N-glycan or may be two or more types of N-glycans.

An IgG antibody according to one embodiment may be prepared by artificially introducing glycan-binding motifs to the Fc region in the IgG antibody so that there are four or more glycan-binding motifs in the Fc region. An IgG antibody according to one embodiment may be obtained as a genetically modified antibody using a mammalian cell as an expression host. In that case, the mammalian cell may be, but is not limited to being, a CHO (Chinese Hamster Ovary) cell, an HEK293 (Human Embryonic Kidney cell 293) cell, etc. Additionally, an IgG antibody according to one embodiment can be prepared by using a plant-based transient expression system, or can be prepared by using a genetically modified plant. As the plant-based transient expression system, for example, the method described in Japanese Patent No. 5015012 can be used. Additionally, techniques for obtaining the antibody as a genetically modified antibody include, but are not limited to, transient expression systems using plasmid vectors, viral vectors, etc. lacking self-replicability, semi-stable expression systems using episomal vectors provided with a nuclear localization signal and having self-replicability, stable expression systems in which a target gene is inserted in the genome of an expression host, etc.
In a host cell, a glycan can be bound to an asparagine residue within a glycan-binding motif in the Fc region of an IgG antibody via glycosyltransferases such as N-acetylglucosamine transferase, mannose transferase, galactose transferase, or fucose transferase.

Whether or not a glycan is bound to the IgG antibody can be checked by liquid chromatography-mass spectroscopy (hereinafter also referred to as "LC-MS"). In LC-MS, for example, an IgG antibody is first fragmented by means of trypsin, then subjected to the LC-MS. Next, a mass spectrum can be detected from fragments including glycan-binding motifs, in which N-glycans are bound to the glycan-binding motifs.
When doing so, the percentage of the total peak area of the mass spectrum from said fragments relative to the total peak area of the mass spectrum from fragments containing the glycan-binding motifs (including fragments in which N-glycans are not bound to said glycan-binding motifs) may be calculated, thereby determining the percentage of glycan-binding motifs to which N-glycans are bound among said glycan-binding motifs.

An insoluble particle according to one embodiment contains a particulate carrier and an IgG antibody to the target antigen, wherein the IgG antibody is carried on the particulate carrier and has four or more N-glycans bound to the Fc region.

As the above-mentioned antibody contained in the insoluble particle, it is possible to use, in addition to an IgG antibody according to one embodiment mentioned above, a human IgG in which four or more N-glycans are bound to the Fc region. The human IgG is preferably human IgG1, human IgG2, and human IgG4 in which a glycan-binding motif is introduced to the Fc region. The mode of binding of the N-glycan to the Fc region of the human IgG is preferably similar to that of an IgG antibody according to one embodiment mentioned above. The production of the human IgG and check regarding whether a glycan is bound to the human IgG may be performed in a manner similar to that for an IgG antibody according to one embodiment mentioned above.

In human IgG, the Fc region in the IgG antibody has 90% or higher identity with the amino acid sequence of SEQ ID NO: 10, 12, or 14. Said amino acid sequences are preferably such that: in SEQ ID NO: 10, a 180-th residue is an asparagine residue, a 181-st residue is an amino acid residue other than proline, a 182-nd residue is a serine residue or a threonine residue, a 307-th residue is an asparagine residue, a 308-th residue is an amino acid residue other than proline, and a 309-th residue is a serine residue or a threonine residue; in SEQ ID NO: 12, a 176-th residue is an asparagine residue, a 177-th residue is an amino acid residue other than proline, a 178-th residue is a serine residue or a threonine residue, a 303-rd residue is an asparagine residue, a 304-th residue is an amino acid residue other than proline, and a 305-th residue is a serine residue or a threonine residue; or in SEQ ID NO: 14, a 177-th residue is an asparagine residue, a 178-th residue is an amino acid residue other than proline, a 179-th residue is a serine residue or a threonine residue, a 304-th residue is an asparagine residue, a 305-th residue is an amino acid residue other than proline, and a 306-th residue is a serine residue or a threonine residue. In the preferable embodiments mentioned above, it is more preferable for glycan-binding motifs to be absent aside from the180-th to 182-nd residues and the 307-th to 309-th residues in SEQ ID NO: 10, aside from the 176-th to 178-th residues and the 303-rd to 305-th residues in SEQ ID NO: 12, or aside from the 177-th to 179-th residues and the 304-th to 306-th residues in SEQ ID NO: 14.

In this case, SEQ ID NO: 10 is the amino acid sequence (SEQ ID NO: 9) of the Fc region in human IgG1 in which the serine residue at the 307-th position has been substituted by an asparagine residue. In SEQ ID NOs: 9 and 10, the 180-th to 182-nd residues are located in the second constant domain, and the 307-th to 309-th residues are located in the third constant domain. SEQ ID NO: 9 has a glycan-binding motif at the 180-th to 182-nd residues, and SEQ ID NO: 10 has glycan-binding motifs at the 180-th to 182-nd residues and at the 307-th to 309-th residues.

SEQ ID NO: 12 is the amino acid sequence (SEQ ID NO: 11) of the Fc region in human IgG2 in which the serine residue at the 303-rd position has been substituted by an asparagine residue. In SEQ ID NOs: 11 and 12, the 176-th to 178-th residues are located in the second constant domain, and the 303-rd to 305-th residues are located in the third constant domain. SEQ ID NO: 11 has a glycan-binding motif at the 176-th to 178-th residues, and SEQ ID NO: 12 has glycan-binding motifs at the 176-th to 178-th residues and at the 303-rd to 305-th residues.

SEQ ID NO: 14 is the amino acid sequence (SEQ ID NO: 13) of the Fc region in human IgG4 in which the serine residue at the 304-th position has been substituted by an asparagine residue. In SEQ ID NOs: 13 and 14, the 177-th to 179-th residues are located in the second constant domain, and the 304-th to 306-th residues are located in the third constant domain. SEQ ID NO: 13 has a glycan-binding motif at the 177-th to 179-th residues, and SEQ ID NO: 14 has glycan-binding motifs at the 177-th to 179-th residues and at the 304-th to 306-th residues.

The target antigen may be, but is not limited to being, for example: a protein marker such as a CRP (C-reactive protein), a prostate-specific antigen, ferritin, β-2 microglobulin, myoglobin, hemoglobin, albumin, or creatinine; an immunoglobulin such as IgG, IgE, IgA, or IgM; various types of tumor markers; a lipoprotein such as LDL, HDL, or TG; a viral antigen such as influenza A virus, influenza B virus, RS virus (RSV), rhinovirus, rotavirus, norovirus, adenovirus, astrovirus, HAV, HBs, HCV, HIV, or EBV; a bacterial antigen such as *Chlamydia trachomatis*, *Streptococcus pyogenes*, *Bordetella pertussis*, *Helicobacter pylori*, *Leptospira*, *Treponema pallidum*, *Toxoplasma gondii*, *Borrelia*, *Legionella*, *Bacillus anthracis*, or MRSA; a toxin produced by bacteria, etc.; a mycoplasma lipid antigen; a peptide hormone such as a human brain natriuretic peptide (hereinafter also referred to as "human BNP") or human chorionic gonadotropin; a steroid such as a steroid hormone; a bioactive amine such as epinephrine or morphine; a vitamin such as the vitamin B group; prostaglandins; an antibiotic such as tetracycline; an agrochemical; an environmental hormone, etc.

The particulate carrier may be a particle such as a latex particle, a ceramic particle, an alumina particle, a silica-alumina particle, or a carbon black particle. Among these particles, a latex particle is preferable. The material of the latex may be, for example, polystyrene, divinylbenzene, etc., and is preferably polystyrene. The average particle diameter of the particulate carrier can be set to be 0.1 to 5 µm. The particle diameter of the particulate carrier can be measured by a dynamic light scattering method. The "average particle diameter" in the present specification means the particle diameter (median diameter) when the cumulative value, from smaller particle diameters, reaches 50% of the total in a volume-based particle diameter distribution curve obtained by a dynamic light scattering method.

The particulate carrier and the IgG antibody in which four or more N-glycans are bound to the Fc region can be bound by using a common technique, for example, a physical adsorption method, a chemical binding method, etc.

A target antigen measurement method according to one embodiment involves using the above-mentioned insoluble particle. This measurement method can be performed in order to qualitatively evaluate whether or not the target antigen is present in a test sample or to quantitatively evaluate the concentration of the target antigen in a test sample.

In one embodiment, the above-mentioned measurement method includes a step of bringing the above-mentioned insoluble particle into contact with a test sample that may contain a target antigen, and a step of measuring an agglutination reaction of the above-mentioned insoluble particle due to an antigen-antibody reaction between the target antigen and an antibody contained in the insoluble particle. As the insoluble particle, a particle that has already been prepared may be used, or the insoluble particle may be prepared at the time of measurement by binding the particulate carrier with the IgG antibody in which four or more N-glycans are bound to the Fc region.

The insoluble particles can be brought into contact with a test sample that may contain the target antigen by mixing the test sample with a suspension including the insoluble particles. By bringing these into contact with each other, interactions between the target antigen included in the test sample and the antibody carried on the particulate carrier cause the insoluble particles to agglutinate, changing the absorbance of the suspension. The change (endpoint method) or the rate of change (rate method) in this absorbance is measured. As the measurement, a turbidimetric method or a colorimetric method is used. For example, from outside a cell, light in the visible light to the near-infrared range, normally 300 nm to 1000 nm, preferably 500 nm to 900 nm, is radiated and the change in the absorbance or the change in the intensity of scattered light is detected, thereby measuring the agglutination reaction of the insoluble particles.

The period of time during which the agglutination reaction is performed can be set to be, but is not limited to being, from 1 minute to 30 minutes, and is preferably from 1 minute to 10 minutes. The temperature at which the agglutination reaction is performed can be set to be, but is not limited to being, from 30 °C to 40 °C, or from 33 to 37 °C.

Multiple reference samples including the target antigen to be measured at various known concentrations are prepared, and the change or the rate of change of the absorbance is measured by the above-mentioned method for these reference samples. A calibration curve is drawn by plotting the concentration of the antigen to be measured in the reference samples on the horizontal axis and the measured change or rate of change in absorbance on the vertical axis. By using the same method to measure the change or the rate of change in the absorbance in an unknown test sample and fitting the measurement results to the above-mentioned calibration curve, the target antigen in the test sample can be quantitatively evaluated. Additionally, a threshold value for the change or the rate of change in the absorbance can be set in advance, such that when the threshold value is exceeded, the target antigen can be qualitatively evaluated as being present in the test sample.

Although the test sample is not particularly limited as long as it contains the target antigen, examples include body fluids such as blood, serum, plasma, urine, stool, saliva, tissue fluid, cerebrospinal fluid, swab fluid, etc., or dilutions thereof. Blood, serum, plasma, urine, stool, cerebrospinal fluid or dilutions thereof are preferable.

A target antigen measurement kit according to one embodiment includes the above-mentioned insoluble particle. The target antigen measurement kit can be used for qualitatively evaluating whether or not the target antigen is present in a test sample, or for quantitatively evaluating the concentration of the target antigen in the test sample. More specifically, it can be used in the above-mentioned target antigen measurement method.

In one embodiment, the target antigen measurement kit may be in a form containing a particulate carrier and an IgG antibody in which four or more N-glycans are bound to the Fc region, these being bound to each other at the time of measurement to prepare a particulate carrier carrying the IgG antibody in which four or more N-glycans are bound to the Fc region.

The target antigen measurement kit may further include target antigens for use as a positive control or to create a calibration curve, and may include a buffer solution for diluting the test sample, a buffer solution for mixing the insoluble particle with the test sample, a buffer solution for binding the particulate carrier with the IgG antibody in which four or more N-glycans are bound to the Fc region, etc.

### EXAMPLES

Hereinafter, the present invention will be explained in further detail based on examples. However, the present disclosure is not limited to these examples.

### <Preparation of pre-modification IgG1 antibodies>

Two types of plasmids (produced in-house) for expressing anti-human brain natriuretic peptide antibodies (hereinafter also referred to as "anti-human BNP antibodies"), which were mouse-derived IgG1 antibodies, were respectively used to transform CHO (Chinese Hamster Ovary) cells, and after the pre-modification IgG1 antibodies were expressed, a Protein A (GE) column was used to purify the culture supernatants, thereby obtaining two types of pre-modification IgG1 antibodies. The above-mentioned two types of pre-modification IgG1 antibodies recognize different epitopes in human BNP.

### <Preparation of IgG1 antibodies with four N-glycans bound to Fc region>

IgG1 antibodies in which four N-glycans were bound to the Fc region (hereinafter also referred to as "glycosylated antibodies") were prepared by artificially introducing glycan-binding motifs Asn-X-Ser/Thr (X being an arbitrary amino acid; hereinafter also referred to as "glycan-binding motifs") to the Fc regions (SEQ ID NO: 1) of the anti-human BNP antibodies that were mouse-derived IgG1 antibodies. Specifically, the above-mentioned two types of plasmids (produced in-house) for expressing the anti-human BNP antibodies that were mouse-derived IgG1 antibodies were prepared, and a mutation was introduced in each antibody by PCR to substitute Asn for Thr at the 424-th position by Eu numbering (the 301-st position in SEQ ID NO: 1), thereby obtaining two types of plasmids for expressing glycosylated antibodies. The Fc regions (SEQ ID NO: 2) of the glycosylated antibodies had two pairs of glycan-binding motifs, i.e., Asn-Ser-Thr (positions 174 to 176) and Asn-Cys-Ser (positions 301 to 303). Asn-Ser-Thr (positions 174 to 176) is located in the second constant domain and Asn-Cys-Ser (positions 301 to 303) is located in the third constant domain.

Each of the two types of plasmids that were obtained was used to transform a CHO (Chinese Hamster Ovary) cell, and after glycosylated antibodies were expressed, a Protein A (GE) column was used to purify the culture supernatants, thereby obtaining two types of glycosylated antibodies.

The two types of glycosylated antibodies were checked regarding whether N-glycans were bound to the artificially introduced glycan-binding motifs by liquid chromatography-mass spectroscopy (hereinafter also referred to as "LC-MS") of peptide fragments (hereinafter also referred to as "mutant peptide fragments") of the glycosylated antibodies including Asn at the 424-th position by Eu numbering. Specifically, first, each of the two types of glycosylated antibodies was sequentially heat-treated using a RapiGest SF solution (Waters Corp.), a dithiothreitol solution, and an iodoacetamide solution, then subjected to a heat treatment overnight in a trypsin solution, and finally treated with formic acid for fragmentation. Next, the fragmented glycosylated antibodies were analyzed by LC-MS to obtain the total peak areas of the mass spectra from the mutant peptide fragments, and the total peak areas of the mass spectra from mutant peptide fragments in which N-glycans were bound to the Asn at the 424-th position by Eu numbering among said mutant peptide fragments. Furthermore, by calculating the percentages of the total peak areas of the latter with respect to the total peak areas of the former, the percentages of the Asn to which N-glycans were bound among the above-mentioned Asn were determined. The above-mentioned percentages were respectively 99.6% and 100% for the two types of glycosylated antibodies. Thus, the results indicated that N-glycans were bound to the Asn at the 424-th position by Eu numbering in the Fc regions of almost all of the glycosylated antibodies.

### <Preparation of antibody-immobilized polystyrene beads>

Two types of pre-modification anti-human BNP antibody solutions and two types of glycosylated antibody solutions were respectively mixed with polystyrene beads (latex particles, average particle diameter 0.3 µm), and allowed to react for 1 hour at 25 °C. Thereafter, centrifugation was performed and the supernatants were removed, after which blocking was performed by adding 1% BSA solutions, thereby obtaining antibody-immobilized polystyrene beads respectively for the two types of pre-modification anti-human BNP antibodies and the two types of glycosylated antibodies. Thereafter, centrifugation was performed and the supernatants were removed, after which dispersion solutions containing 5 mM 3-morpholinopropanesulfonic acid (pH 7.0), 5 mass% of sucrose, and 0.4 mass% of BSA were added and ultrasonic treatments were performed to cause dispersion. Equal amounts of the antibody-immobilized polystyrene beads with the two types of pre-modification anti-human BNP antibodies were mixed with each other and equal amounts of the antibody-immobilized polystyrene beads with the two types of glycosylated antibodies were mixed with each other to adjust the final concentrations to be 0.125 wt%, thereby obtaining antibody-immobilized polystyrene beads for each of the pre-modification anti-human BNP antibodies and the glycosylated antibodies.

### <Preparation of stabilizing solution>

A stabilizing solution containing 120 mM Tris (pH 8.5), 300 mM KCl, and 0.4 mass% of Lipidure^{®}-BL405 (NOF Corp.) was prepared.

### <Evaluation of reactivity of antibody-immobilized polystyrene beads>

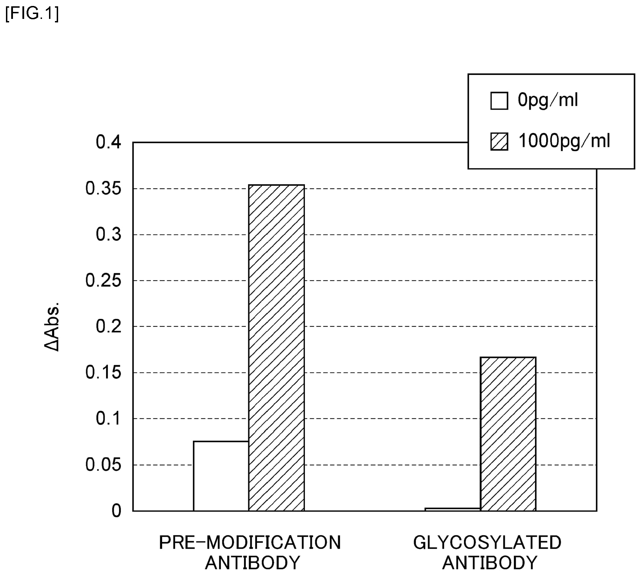

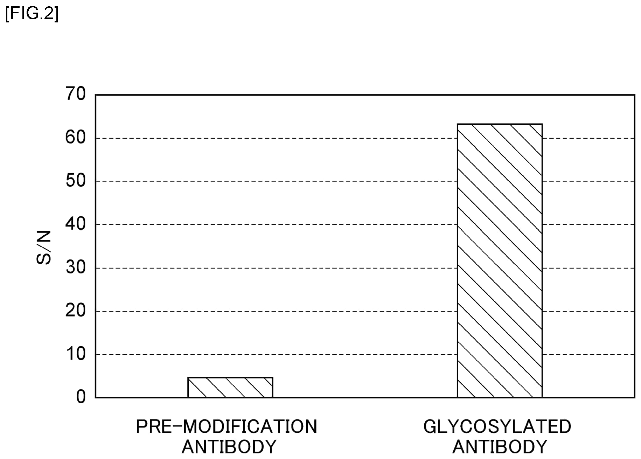

The effects of the glycosylated antibodies in the presence and absence of human BNP were investigated by a two-reagent latex agglutination immunoassay comprising the prepared stabilizing solution and the antibody-immobilized polystyrene bead solutions. Specifically, first, 100 µL of the prepared stabilizing solution were added to 10 µL of a diluting solution (2 mass% BSA solution) containing 1000 pg/ml of human BNP or not containing human BNP and mixed, then placed at rest for 5 minutes at 37 °C. Thereafter, 30 µL of prepared antibody-immobilized polystyrene bead solutions with the pre-modification anti-human BNP antibodies or the glycosylated antibodies were further added and stirred, then allowed to react for 5 minutes at 37 °C. The absorbance for 5 minutes after adding the antibody-immobilized polystyrene bead solution was measured continuously, using a clinical chemistry analyzer TBA-120F, with light measurement points set at 21-23 and 31-33, and with the main measurement wavelength set at 628 nm, and the change in absorbance during the 5 minutes was calculated. The results are indicated in FIG. 1. In FIG. 1, "ΔAbs." indicates the above-mentioned change in absorbance. Additionally, the results of evaluation of the ratios (hereinafter also referred to as "S/N") of the change in absorbance with1000 pg/ml of human BNP to the change in absorbance with 0 pg/ml of human BNP are indicated in FIG. 2.

From FIG. 1, it was confirmed that, when a glycosylated antibody was used, the value with 0 pg/ml of human BNP was largely reduced in comparison with the case in which the pre-modification anti-human BNP antibody was used. This is inferred to be due to the fact that the antibody-immobilized polystyrene beads with the glycosylated antibodies had improved dispersibility due to the presence of the N-glycans, thereby suppressing non-specific agglutination of the antibody-immobilized polystyrene beads. Additionally, FIG. 2 indicates that, in the case in which glycosylated antibodies were used, the above-mentioned S/N was significantly higher than in the case in which pre-modification anti-human BNP antibodies were used. The above indicates that, by having four or more N-glycans bound to the Fc regions of the IgG1 antibodies, higher performance is possible, such as the suppression of non-specific reactions in latex agglutination immunoassays on antibody-immobilized polystyrene beads.

### <Evaluation of non-specific reactions in plasma specimens>

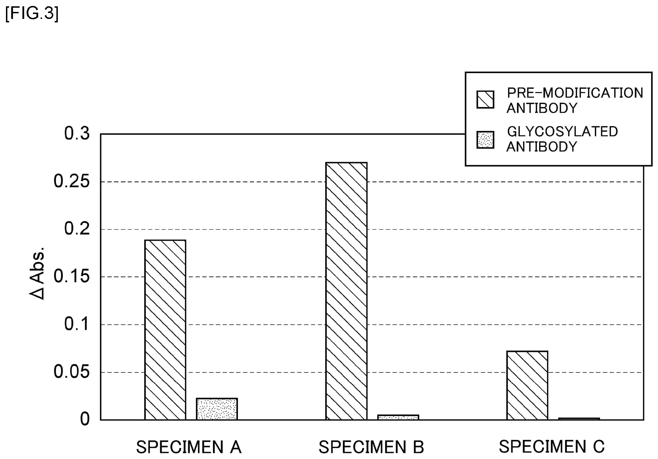

The changes in absorbance over 5 minutes were calculated by a method similar to that described in the above-mentioned "Evaluation of reactivity of antibody-immobilized polystyrene beads", except that three types of specimens (specimens A to C) from plasma not containing (equal to or less than the detection limit) human BNP were used instead of the diluting solutions (2 mass% BSA solutions) containing 1000 pg/ml of human BNP and not containing human BNP. The results are indicated in FIG. 3. In FIG. 3, "ΔAbs." indicates the above-mentioned change in absorbance.

From FIG. 3, in all of specimens A to C, non-specific reactions were detected in the pre-modification anti-human BNP antibodies, yet the non-specific reactions were significantly suppressed in the glycosylated antibodies. This suggested that the glycosylated antibodies have the effect of suppressing non-specific reactions even with respect to actual specimens such as specimens from plasma.

### <Antigen-binding activity evaluation of antibodies>

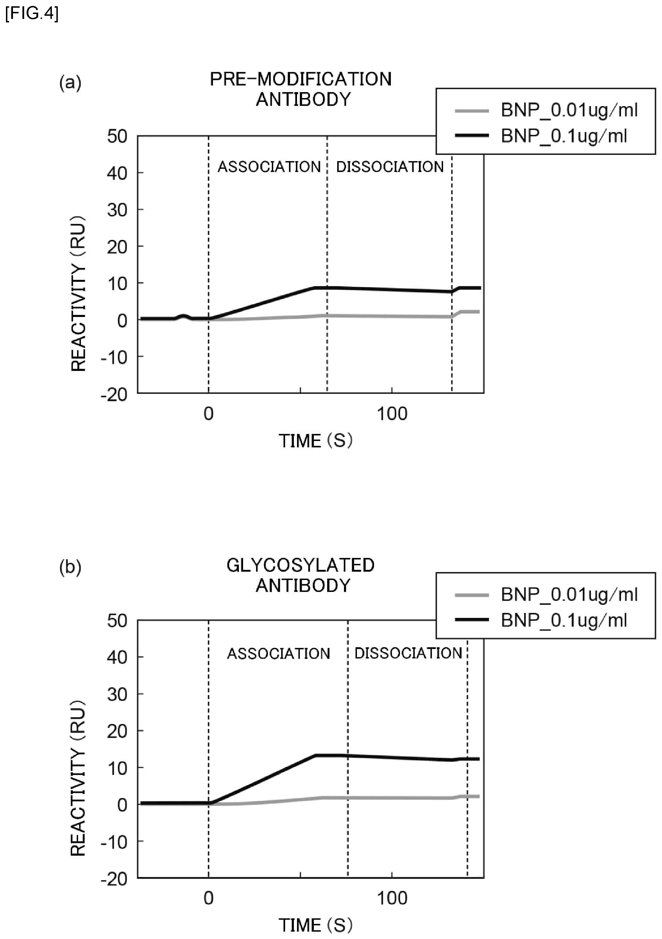

In order to check whether the antigen-binding activity of the glycosylated antibodies was not reduced by the binding of the N-glycans, the antigen-binding activities of the glycosylated antibodies and the pre-modification anti-human BNP antibodies were evaluated by means of antibody immobilization methods using Biacore^{®} 8K (GE). Specifically, first, each type of antibody was immobilized on a Series S Sensor Chip CM5 in accordance with the manufacturer's protocol to prepare sensor chips for measurement. These sensor chips were used to evaluate binding with 0.01 µg/ml and 0.1 µg/ml human BNP solutions in accordance with the manufacturer's protocol. The results are indicated in FIG. 4. In FIG. 4, the horizontal axis represents time (seconds) and the vertical axis represents reactivity (RU). Flow cells were used to bring the analyte into contact with the sensor chips on which the glycosylated antibody or the pre-modification anti-human BNP antibody were immobilized, an association phase during the time period indicated by "Association" and a dissociation phase during the time period indicated by "Dissociation" in FIG. 4.

From FIG. 4, no difference is observed between the antigen-binding activities of the glycosylated antibodies and the pre-modification anti-human BNP antibodies, thus indicating that the binding of N-glycans does not affect the antigen-binding activity of the antibodies.

## Claims

1. An IgG antibody, other than human IgG, having a Fab region and an Fc region connected to the Fab region, wherein four or more N-glycans are bound to the Fc region.

2. The antibody according to claim 1, wherein two N-glycans among the four or more N-glycans are bound to a second constant domain, and two N-glycans different from the aforementioned two N-glycans among the four or more N-glycans are bound to a third constant domain.

3. The antibody according to claim 2, wherein an amino acid sequence of the Fc region:
(1) has 90% or higher identity with an amino acid sequence of SEQ ID NO: 2, and in the amino acid sequence,
a 174-th residue is an asparagine residue, a 175-th residue is an amino acid residue other than proline, a 176-th residue is a serine residue or a threonine residue, a 301-st residue is an asparagine residue, a 302-nd residue is an amino acid residue other than proline, and a 303-rd residue is a serine residue or a threonine residue;
(2) has 90% or higher identity with an amino acid sequence of SEQ ID NO: 4, and in the amino acid sequence,
a 180-th residue is an asparagine residue, a 181-st residue is an amino acid residue other than proline, a 182-nd residue is a serine residue or a threonine residue, a 307-th residue is an asparagine residue, a 308-th residue is an amino acid residue other than proline, and a 309-th residue is a serine residue or a threonine residue;
(3) has 90% or higher identity with an amino acid sequence of SEQ ID NO: 6, and in the amino acid sequence,
a 185-th residue is an asparagine residue, a 186-th residue is an amino acid residue other than proline, a 187-th residue is a serine residue or a threonine residue, a 312-th residue is an asparagine residue, a 313-th residue is an amino acid residue other than proline, and a 314-th residue is a serine residue or a threonine residue;
(4) has 90% or higher identity with an amino acid sequence of SEQ ID NO: 8, and in the amino acid sequence,
a 179-th residue is an asparagine residue, a 180-th residue is an amino acid residue other than proline, a 181-st residue is a serine residue or a threonine residue, a 306-th residue is an asparagine residue, a 307-th residue is an amino acid residue other than proline, and a 308-th residue is a serine residue or a threonine residue; or
(5) has 90% or higher identity with an amino acid sequence of SEQ ID NO: 16, and in the amino acid sequence,
a 173-rd residue is an asparagine residue, a 174-th residue is an amino acid residue other than proline, a 175-th residue is a serine residue or a threonine residue, a 300-th residue is an asparagine residue, a 301-st residue is an amino acid residue other than proline, and a 302-nd residue is a serine residue or a threonine residue.

4. An insoluble particle containing a particulate carrier and an IgG antibody to a target antigen, the IgG antibody being carried on the particulate carrier, wherein:
the antibody has a Fab region and an Fc region connected to the Fab region; and
four or more N-glycans are bound to the Fc region.

5. The insoluble particle according to claim 4, wherein, in the antibody, two N-glycans among the four or more N-glycans are bound to a second constant domain, and two N-glycans different from the aforementioned two N-glycans among the four or more N-glycans are bound to a third constant domain.

6. The insoluble particle according to claim 4, wherein the particulate carrier is a latex particle.

7. The insoluble particle according to claim 5, wherein an amino acid sequence of the Fc region in the antibody:
(1) has 90% or higher identity with an amino acid sequence of SEQ ID NO: 2, and in the amino acid sequence,
a 174-th residue is an asparagine residue, a 175-th residue is an amino acid residue other than proline, a 176-th residue is a serine residue or a threonine residue, a 301-st residue is an asparagine residue, a 302-nd residue is an amino acid residue other than proline, and a 303-rd residue is a serine residue or a threonine residue;
(2) has 90% or higher identity with an amino acid sequence of SEQ ID NO: 4, and in the amino acid sequence,
a 180-th residue is an asparagine residue, a 181-st residue is an amino acid residue other than proline, a 182-nd residue is a serine residue or a threonine residue, a 307-th residue is an asparagine residue, a 308-th residue is an amino acid residue other than proline, and a 309-th residue is a serine residue or a threonine residue;
(3) has 90% or higher identity with an amino acid sequence of SEQ ID NO: 6, and in the amino acid sequence,
a 185-th residue is an asparagine residue, a 186-th residue is an amino acid residue other than proline, a 187-th residue is a serine residue or a threonine residue, a 312-th residue is an asparagine residue, a 313-th residue is an amino acid residue other than proline, and a 314-th residue is a serine residue or a threonine residue;
(4) has 90% or higher identity with an amino acid sequence of SEQ ID NO: 8, and in the amino acid sequence,
a 179-th residue is an asparagine residue, a 180-th residue is an amino acid residue other than proline, a 181-st residue is a serine residue or a threonine residue, a 306-th residue is an asparagine residue, a 307-th residue is an amino acid residue other than proline, and a 308-th residue is a serine residue or a threonine residue;
(5) has 90% or higher identity with an amino acid sequence of SEQ ID NO: 10, and in the amino acid sequence,
a 180-th residue is an asparagine residue, a 181-st residue is an amino acid residue other than proline, a 182-nd residue is a serine residue or a threonine residue, a 307-th residue is an asparagine residue, a 308-th residue is an amino acid residue other than proline, and a 309-th residue is a serine residue or a threonine residue;
(6) has 90% or higher identity with an amino acid sequence of SEQ ID NO: 12, and in the amino acid sequence,
a 176-th residue is an asparagine residue, a 177-th residue is an amino acid residue other than proline, a 178-th residue is a serine residue or a threonine residue, a 303-rd residue is an asparagine residue, a 304-th residue is an amino acid residue other than proline, and a 305-th residue is a serine residue or a threonine residue;
(7) has 90% or higher identity with an amino acid sequence of SEQ ID NO: 14, and in the amino acid sequence,
a 177-th residue is an asparagine residue, a 178-th residue is an amino acid residue other than proline, a 179-th residue is a serine residue or a threonine residue, a 304-th residue is an asparagine residue, a 305-th residue is an amino acid residue other than proline, and a 306-th residue is a serine residue or a threonine residue; or
(8) has 90% or higher identity with an amino acid sequence of SEQ ID NO: 16, and in the amino acid sequence,
a 173-rd residue is an asparagine residue, a 174-th residue is an amino acid residue other than proline, a 175-th residue is a serine residue or a threonine residue, a 300-th residue is an asparagine residue, a 301-st residue is an amino acid residue other than proline, and a 302-nd residue is a serine residue or a threonine residue.

8. A target antigen measurement kit including the insoluble particle according to any one of claims 4 to 7, or an antibody and a particulate carrier for preparing the insoluble particle according to any one of claims 4 to 7.

9. A target antigen measurement method wherein the insoluble particle according to any one of claims 4 to 7 is used.

10. A target antigen measurement method including:
a step of bringing the insoluble particle according to any one of claims 4 to 7 into contact with a test sample that may contain a target antigen; and
a step of measuring an agglutination reaction of the insoluble particle due to an antigen-antibody reaction between the target antigen and an antibody contained in the insoluble particle.
